(19) **Europäisches Patentamt** / **European Patent Office** / **Office européen des brevets**

(11) **EP 2 367 576 B1**

(12) **EUROPEAN PATENT SPECIFICATION**

(45) Date of publication and mention of the grant of the patent:
**20.02.2019 Bulletin 2019/08**

(21) Application number: **09761017.4**

(22) Date of filing: **19.11.2009**

(51) Int Cl.:
*A61L 27/54* *(2006.01)*      *A61L 31/18* *(2006.01)*
*A61K 49/00* *(2006.01)*      *A61K 49/18* *(2006.01)*
*A61K 51/04* *(2006.01)*      *A61L 27/58* *(2006.01)*
*A61L 31/14* *(2006.01)*

(86) International application number:
**PCT/IB2009/055168**

(87) International publication number:
**WO 2010/058361 (27.05.2010 Gazette 2010/21)**

(54) **METHOD FOR THE PRODUCTION OF SCAFFOLDS FOR TISSUE ENGINEERING, COMPRISING THE USE OF AN ANCHORING UNIT, AND SCAFFOLD PRODUCED THEREWITH**

VERFAHREN ZUR HERSTELLUNG VON GERÜSTEN FÜR DIE GEWEBETECHNIK, DAS DIE VERWENDUNG VON ANKEREINHEITEN UMFASST, UND DAMIT HERGESTELLTES GERÜST

PROCÉDÉ DE FABRICATION D'ÉCHAFAUDAGES MOLÉCULAIRES POUR L'INGÉNIERIE TISSULAIRE UTILISANT UNE UNITÉ D'ANCRAGE, ET ÉCHAFAUDAGE MOLÉCULAIRE FABRIQUÉ PAR CE PROCÉDÉ

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **24.11.2008 EP 08169783**

(43) Date of publication of application:
**28.09.2011 Bulletin 2011/39**

(73) Proprietor: **Koninklijke Philips N.V.
5656 AE Eindhoven (NL)**

(72) Inventors:
- **HALTER, David**
  **5656 AE Eindhoven (NL)**
- **KURT, Ralph**
  **5656 AE Eindhoven (NL)**
- **PEETERS, Emiel**
  **5656 AE Eindhoven (NL)**
- **PENTERMAN, Roel**
  **5656 AE Eindhoven (NL)**
- **BROER, Dirk J.**
  **5656 AE Eindhoven (NL)**
- **LAMERICHS, Rudolf M. J. N.**
  **5656 AE Eindhoven (NL)**

(74) Representative: **Kroeze, Johannes Antonius
Philips Intellectual Property & Standards
High Tech Campus 5
5656 AE Eindhoven (NL)**

(56) References cited:
**WO-A2-2007/039858      WO-A2-2007/039864
US-A1- 2006 204 445      US-A1- 2007 014 729**

- **AZOULAY M ET AL: "A new drug-release method using the Staudinger ligation" BIOORGANIC & MEDICINAL CHEMISTRY LETTERS, PERGAMON, ELSEVIER SCIENCE, GB LNKD-DOI:10.1016/J.BMCL.2006.03.073, vol. 16, no. 12, 15 June 2006 (2006-06-15) , pages 3147-3149, XP025106197 ISSN: 0960-894X [retrieved on 2006-06-15]**
- **PRESCHER J A ET AL: "Chemical remodelling of cell surfaces in living animals" NATURE, NATURE PUBLISHING GROUP, LONDON, GB LNKD- DOI:10.1038/NATURE02791, vol. 430, no. 7002, 19 August 2004 (2004-08-19), pages 873-877, XP002363185 ISSN: 0028-0836**

- **VAN LIESHOUT M I ET AL: "Electrospinning versus knitting: two scaffolds for tissue engineering of the aortic valve" JOURNAL OF BIOMATERIALS SCIENCE. POLYMER EDITION, VSP, UTRECHT, NL LNKD-DOI:10.1163/156856206774879153, vol. 17, no. 1-2, 1 January 2006 (2006-01-01), pages 77-89, XP008121958 ISSN: 0920-5063**

**Description**

FIELD OF THE INVENTION

The present invention is related to scaffold materials and scaffolds for tissue engineering and organ engineering. More particularly, the present invention is related to the labelling of scaffold materials and scaffolds, which serve as contrast agents for medical imaging means, like CT, MRI, X-Ray, ultrasound, scintigraphy and the like.

BACKGROUND OF THE INVENTION

Tissue engineering is a relatively young discipline which aims at producing, in the laboratory, tissues, or organs, which may then be used to repair, or replace, defective tissues, or organs, of a patient.

In many cases, the said tissues, or organs, are being produced with help of a scaffold, this being a three-dimensional matrix which the cells use as basis for their growth, and division, either *in vitro* or *in vivo.* Such scaffold needs to mimic the *in vivo* milieu, and enable cells to influence their own microenvironment. In order to do so, it needs to allow cell attachment and migration, deliver and retain cells and biochemical factors, enable diffusion of vital cell nutrients and expressed products, and exert certain mechanical and biological influences to modify the behaviour of the cells.

To provide for a proper functioning of the implanted tissue it is desirable to be able to visually control the actual state of the scaffold. This can be of particular importance for monitoring the continuous bio-degradation of the scaffold and to assess the structural and mechanical properties during this degradation process. However, the cells growing on the scaffold as well as the scaffolds itself provide little, or even no, contrast compared to the surrounding tissues in clinically relevant imaging modalities such as CT, MRI, X-Ray, scintigraphy and/or Ultrasound imaging, and can therefore hardly be visualized.

US2006/0204445 discloses a matrix having a three-dimensional ultrastructure of interconnected fibers and pores to permit cell attachment, and further comprising an image enhancing agent. Said image enhancing agent is an MRI imaging label based on lanthanides and/or transition elements. The matrix comprises biomaterials, such as collagen, elastin, fibrous proteins and/or polysaccharieds, and/or a synthetic polymer, and is for example produced by electrospinning. The image enhancing agents are incorporated within, or on, the scaffold matrix before seeding with cells. When the colonized scaffold forms a tissue layer of cells and is ready for use, the growth, development, and remodeling of the artificial tissue can be monitored using the incorporated agents.

This approach, however, has some serious drawbacks, one of them being the fact that the type of label which is used must be invariably determined at the time the scaffold is produced. Under some circumstances, a given label may however turn out unsuitable, e.g., for a given imaging device, or because it elicits an immune response in a given patient. Furthermore, label bleaching may occur.

Another disadvantage may arise from permanent *in-situ* release of the respective label, or its matabolic products, once the scaffold, or the tisse or organ, is implanted, namely due to cleavage of the respective bindings, and/or metabolic degradation of the labels, in a physiologic environment, e.g., by effect of ubiquituos esterases and electrolytes.

Yet another disadvantage may arise from the fact that the scaffold is equipped with the labelling agent prior to cell colonization. As suspended cells, which are meant to settle down on the scaffold, and build up the desired tissue, or organ, are extremely delicate towards compounds as radionuclides, heavy metals, charged entities, salts and the like (which are being used frequently as labelling agents, see table 1), the presence of the latter might impair the colonization of the scaffold with cells, or division of cells which have just colonized the scaffold.

WO 2007/039858 A2 discloses the use of the [3+2] azide-alkyne cycloaddition as biorthogonal reaction in targeted molecular imaging and therapy.

SUMMARY OF THE INVENTION

It is the object of the present invention to provide a process for labelling a scaffold for tissue engineering, or an engineered tissue or organ, which overcomes at least some of the above mentioned disadvantages.

It is another object of the present invention to provide a process for labelling a scaffold for tissue engineering, or an engineered tissue or organ, which provides for more flexibility in terms of imaging options than the methods from the prior art.

It is another object of the present invention to provide a process for labelling a scaffold for tissue engineering, or an engineered tissue or organ, which allows a patient specific individualization.

It is another object of the present invention to provide a process for labelling a scaffold for tissue engineering which reduces the risk of impairing scaffold colonization.

These objects are achieved by the method as set forth in the independent claims. The dependent claims indicate preferred embodiments. In this context it is noteworthy to mention that all ranges given in the following are to be understood as that they include the values defining these ranges.

BRIEF DESCRIPTION OF THE DRAWINGS

Additional details, features, characteristics and advantages of the object of the invention are disclosed in the subclaims, the figures and the following description of the respective figure and examples, which, in an exemplary fashion, show preferred embodiments according to the invention. It is to be understood that the examples are by no means meant as to limit the scope of the invention.

Fig. 1 shows a scaffold for an artificial heart valve.
Fig. 2a shows some elements of the invention.
Fig. 2b shows a preferred embodiment of the invention in which the attachment of the anchoring units takes place prior to polymerization of the monomers.
Fig. 3 shows a process in which anchoring units not covalently bound to a monomer are mixed with polymerized scaffold matter.
Fig. 4a shows the so-called Staudinger ligation.
Fig. 4b shows the so-called click-reaction.
Fig. 5a shows a single-stranded oligonucleotide bound to an anchoring unit.
Fig. 5b shows two double-stranded oligonucleotides with sticky ends, which can be used as binding agents according to the invention.
Fig. 6 shows different examples of other nucleotides serving as binding agents according to the invention.
Figs. 7 - 9 show the steps of labelling a scaffold with click chemistry comprising a covalently bound anchoring unit.
Figs. 10 - 12 show the steps of labelling a scaffold by means of Gd-labelled oligonucleotides.
Fig. 13 shows an embodiment in which several oligonucleotide binding agents are linked to a spherical bead.

DETAILED DESCRIPTION OF EMBODIMENTS

According to the invention, a method for the production of scaffold materials and/or scaffolds for tissue and/or organ engineering is provided, said method comprising the addition of at least one anchoring unit for a labelling agent, to at least one scaffold material and/or to at least one scaffold, and the method further comprising the step of binding at least one labelling agent to at least one anchoring unit for a labelling agent, wherein the step of binding at least one labelling agent to at least one anchoring unit is carried out by means of a bio-orthogonal chemical reaction.
Basic methods for the production of scaffold materials and/or scaffolds are well known in the art, some of them being described herein below.
The said method gives more flexibility to the labelling process, as it allows to postpone the decision which labelling agent is to be used to a later point of time, and decouples it from the scaffold production process.
This again allows a better patient-specific selection of the labelling agent, in order to account, among others, for potential allergies and the like.
Furthermore, this allows to select the labelling agent in accordance to a potential imaging device. This is particularly useful, as both imaging devices and labelling agents are subject of extensive research and development, in order to achive better images and improve the quality of diagnosis and examination, while reducing costs, side effects in the patient, and environmental problems at the same time. The method according to the invention is thus open to incorporate future labelling agents with better properties as well.
The term "anchoring unit for a labelling agent", as used herein, refers to a component of a multiple-component system, in which the components (i.e., at least the anchoring unit for a labelling agent and a labelling agent) may be attached to one another either covalently or non-covalently. In this multiple-component system, the anchoring unit is attached, or incorporated, to the scaffold, whereas the labelling agent is later attached to the anchoring unit either covalently or non-covalently.
It is provided that the method further comprises the step of binding at least one labelling agent to at least one anchoring unit for a labelling agent.
The labelling agent does, in most cases, comprise an entity which allows binding to the said anchoring unit. This entity is also called "complememtary binding unit" in the following. The complementary binding unit can either be bound to the labelling agent, or it can form part of the labelling agent.
Therefore, the term "labelling agent" is to be understood as

a) either being bound to a separate complementary binding unit, or
b) comprising, as an integral part, a complementary binding unit.

In a preferred embodiment, said labelling agent can be selected from the following table, which is not to be understood as limiting the scope of the present invention to the labelling agents mentioned. Note that agents marked with a superscript integer are radionuclides.

**Table 1**

| Imaging technology | labelling agent | Examples |
|---|---|---|
| X-ray | Barium based agents, *e.g.,* barium sulphate based agents | |
| | iodine based agents | Hyperosmolaric (Gastrolux®, Gastrografin®, Peritrast®). |
| spectral CT | Gadolinium-complexes | |
| | non-ionic Iodine based agents | Ultravist®, Isovist®, Xenetix® |
| single photon emission computed tomography (Spect) | | |
| magnetic resonance imaging (MRI) | Lanthanaide ions and complexes, *e.g.,* Gadolinium-complexes, like Gd-DTP A-BMA | |
| | PEG-coated iron oxide | PEG-Ferron |
| | SPIO/Iron oxide nano particles (silicon coated, sup erp aramagnetic) | |
| | Manganese based agents (Mangan-DPDP) | |
| | Manganese oxide nano particles | |
| | perfluorooctyl Bromide | |
| | bariumsulfate-suspensions | |
| | Iron Oxide | Ferumoxsil |
| | $^{19}$Fluorine containing materials, *e.g.*, perfluorooctyl bromide (PFOB) | |
| sonography | Gas filled microbubbles | |
| positron emission tomography (PET) | $^{11}$Carbon, $^{13}$Nitrogen, $^{15}$Oxygen, $^{18}$Fluorine, $^{19}$Fluorine, all (optionally) organically bound | |
| scintigraphy | $^{99}$Technetium, $^{121}$Iodine, $^{131}$Iodine, $^{201}$Thallium, $^{67}$Gallium, $^{18}$Fluorine, $^{19}$Fluorine, Fluorodeoxyglucose, $^{111}$Indium | |

The step of binding at least one labelling agent to at least one anchoring unit is carried out by means of a bio-orthogonal chemical reaction.

Bio-orthogonal chemical reactions have to meet the following criteria:

(i) they must not have detrimental effects on the survival of the cells grown on the scaffold (they must be biocompatible),
(ii) they need to have a selective reactivity in order to provide a specific binding behavior, and
(iii) they must not have detrimental effects on the survival and function of the tissue or body in which the reaction takes place.

Binding mechanisms capable of building up such bonds comprise, for example, the so-called "Staudinger reaction" (*i.e.,* the combination of an azide with a phosphine or phosphate to produce an iminophosphorane), the "Staudinger Ligation" (*i.e.*, formation of an iminophosphorane through nucleophilic addition of the phosphine at the terminal nitrogen atom of the azide and expulsion of nitrogen, see Saxon *et al.* 2007.), or the so called "click reaction" (*i.e.,* so called "Strain Promoted [3+2] Azide-Alkyne cycloaddition", see Agard *et al.* 2004).

The said binding mechanisms, and binding agents capable of carrying out such mechanism, are for example disclosed in US20080075661A1, WO2007110811A2 and WO2007039864. It is particularly important that these binding mechanisms allow for an *in vivo*-binding (see below) of the labelling agent to the anchoring unit, and thus to the scaffold.

By means of illustration, not by means of limitation, other bio-orthogonal reactions useful in the context of the present invention, and comprised by its scope, are discussed in the following table gives an overview of potential bio-orthogonal reactions mentioned therein.

**Table 2**

| binding agent 1 | complementary binding agent | chemistry | R |
|---|---|---|---|
| protein with a Tetracystein motif (R-CysCysXaaXaaCysCys-R) where Xaa can be any amino acids, but Pro and Gly preferred | Biarsenical ligand | | protein |
| Ketone (R-CO-R) Aldehyde (R-COH) | $H_2NNH$-CO-R $H_2NO$-R | | protein glycane |
| Azide (R-$N_3$) | phosphine group | staudinger ligation | protein, glycane, lipid |
| | terminal alkyne (-C≡CH) cyclic alkyne | click reaction | |

Other bio-orthogonal binding reactions between at least an anchoring unit and at least a labelling agent can be accomplished by biocompatible binding agents.

A particularly preferred combination of these binding agents is a pair of complementary oligonucleotides, which bind to one another under appropriate conditions by base pairing (nucleic acid hybridization).

The terms "nucleic acid", "polynucleotide" and "oligonucleotide" as used herein, refer to, among others, monomers, oligomers and polymers of RNA, DNA, LNA, PNA, Morpholino and other nucleic acid analogues. A peptide nucleic acid (PNA) is an artificially synthesized polymer similar to DNA or RNA which cofeatures backbone composed of repeating N-(2-aminoethyl)-glycine units linked by peptide bonds. A locked nucleic acid (LNA) is a modified RNA nucleotide in which the ribose moiety is modified with an extra bridge connecting the 2' and 4' carbons. Morpholinos are synthetic analogues of DNA which differ structurally from DNA in that while Morpholinos have standard nucleic acid bases, those bases are bound to morpholine rings instead of deoxyribose rings and linked through phosphorodiamidate groups instead of phosphates.

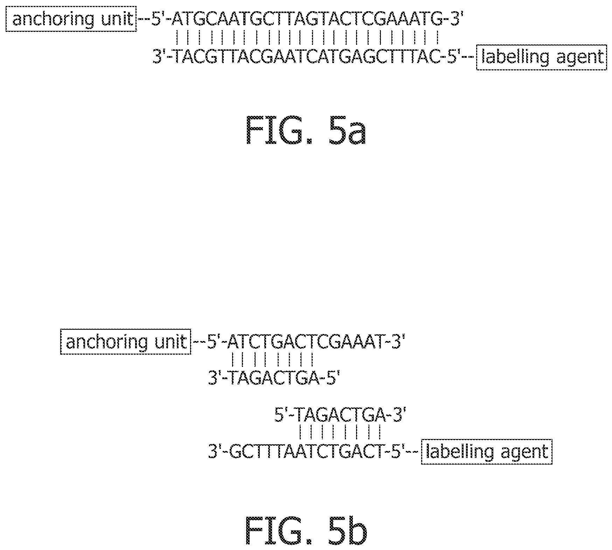

The said oligonucleotides can be single-stranded or, at least in part, double-stranded. In the latter case, for carrying out a binding between a binding agent and its complementary binding agent, the oligonucleotides may have so-called "sticky ends", which are characterized by single strand overhangs. An overhang is a stretch of unpaired nucleotides in the end of a DNA molecule. These unpaired nucleotides can be in either strand, creating either 3' or 5' overhangs. Such sticky ends are shown in Fig. 5.

The said complementary oligonucleotides allow, for example, a controlled binding and cleaving of the labelling agent to the scaffold. These features make the said complementary oligonucleotides particularly useful for *in vivo* binding, as discussed below.

Cleaving can be achieved by using DNA restriction enzymes. As the latter are enzymes that cut double-stranded DNA at specific recognition sequences (known as restriction sites), highly specific cleaving can be obtained by sequence specific design of the respective oligonucleotides. It is preferred, in this context, to select a restriction enzyme, which has

a) low, or at least manageable, toxic, pyrogenic and/or immunogenic potential, and
b) has a restriction site not abundant in the genomic or mitochondrial DNA of the respective patient.

This means that in this approach the same rules apply as if a restriction enzyme were to be used for therapy, *e.g.,* as an anti-virus treatment, where restriction enzymes are preferred which cleave viral nucleotide sequences rather than those of the patient/host. Such approach is for example disclosed in US5523232.

The following table gives an overview of some restriction enzymes and their restriction sites, in order to illustrate the different cleaving options. It has however not been verified if these restrictions enzymes meet the criteria set forth above (i.e., toxicity/immunogenicity and/or alien restriction sites). It is yet to be understood that the skilled person may, from textbooks and scientific literature, may design a restriction site suitable for his purposes, and select a suitable restriction enzyme.

**Table 3**

| restriction enzyme | restriction site | cut | remarks |
|---|---|---|---|
| *Eco*RI | 5'-GAATTC-3'<br>3'-CTTAAG-5' | 5'---G AATTC---3'<br>3'---CTTAA G---5' | |
| *Hinf*I | 5'GANTC-3'<br>3'CTNAG-5' | 5'---G ANTC---3'<br>3'---CTNA G---5' | N = C or G or T or A |
| *Sma*I* | 5'CCCGGG-3'<br>3'GGGCCC-5' | 5'---CCC GGG---3'<br>3'---GGG CCC---5' | |
| *Eco*RII | 5'CCWGG-3'<br>3'GGWCC-5' | 5'--- CCWGG---3'<br>3'---GGWCC ---5' | W = A or T |

Another way of post-imaging release of the labelling agent bound my means of an oligonucleotide is the application of a local temperature increase. This is commonly done by heating the hybridized nucleotides to a temperature of above 85 °C (*i.e.*, melting temperature).

One way to determine the said melting temperature is the so-called Wallace method, which is suitable for oligonucleotides less than 18mers in length. It is being done by counting the frequency of each nucleotide base. The reasoning behind the method is that, because cytosine-guanine pairs form three hydrogen bonds while adenosine and thymine form two hydrogen bonds, the former contribute more to the stability of a double-helix.

The Wallace method is based on the following equation:

$$T_m = 2(A + T) + 3(G + C)$$

The locally focused application of heat, as described above, can for example be accomplished by application of high intensity focused ultrasound (HIFU). The latter is a highly precise medical procedure using high-intensity focused ultrasound to heat (and sometimes destroy) tissue rapidly. Therapeutic ultrasound is a minimally invasive or non-invasive method to deposit acoustic energy into tissue. Conventional applications according to the state of the art include tissue ablation (for tumor treatments, for example) or hyperthermia treatments (low-level heating combined with radiation or chemotherapy). The ultrasound beam can be focused geometrically, for example with a lens or with a spherically curved transducer, or electronically, by adjusting the relative phases of elements in an array of transducers (a "phased array"). By dynamically adjusting the electronic signals to the elements of a phased array, the beam can be steered to different locations, and aberrations due to tissue structures can be corrected. HIFU is in some cases carried out under control of ultrasonography or computerized MRI.

Other ways to apply heat in a locally focused fashion comprise the use of magnetic beads brought into oscillation by a locally applied AC field, or the application of infrared light. The latter is particularly beneficial for the use in scaffolds, or implants, which are located in the periphery of the patient's body (*e.g.,* a nasal cartilage implant).

Furthermore, oligonucleotides provide the option that a given anchoring unit is being labeled with two or more labelling agents. This allows the simultaneous labelling of a scaffold with two or more different labelling agents (for example to allow sequential, or simultaneous, imaging with two or more different imaging means).

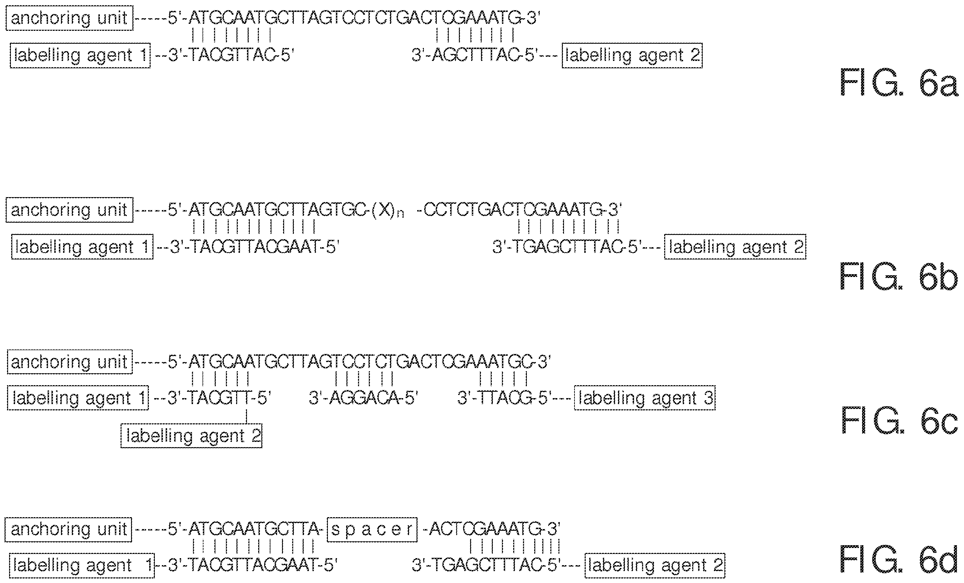

One can for example choose an oligonucleotide with 40 residues (40-mer), together with complementary oligonucleotides being coupled to a labelling agent with 10 residues each (10-mers). In another embodiment, an oligonucleotide can comprise a spacer which separates two sequences from one another. Examples are shown in Fig. 6.

The term "spacer", as used herein, refers to a chemical linker, polymer, peptide and the like that spatially separates different sections of a binding agent, like an oligonucleotide. Preferably, the spacer is selected such that it allows the binding of two or more complementary binding agents in such way that the latter do not interfere with one another.

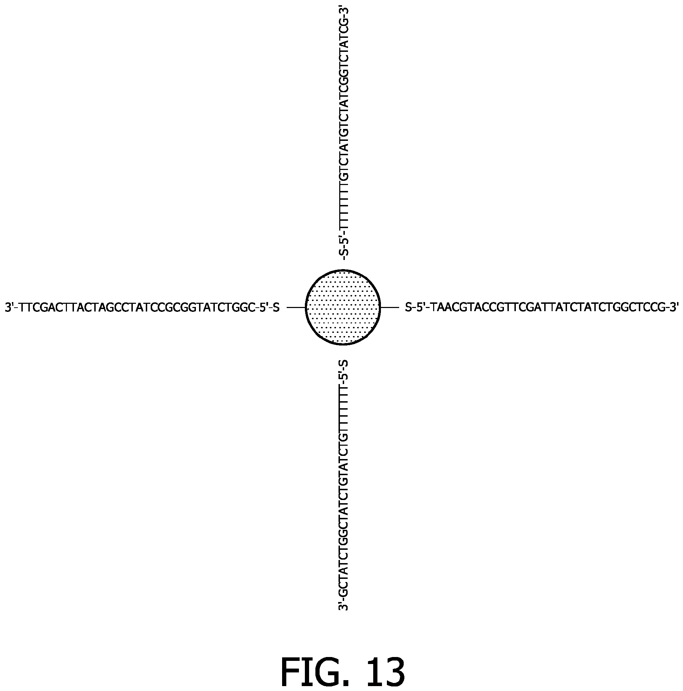

Another option is that several oligonucleotides are linked to a spherical bead incorporated in the scaffold material, as shown in Fig. 13. In this embodiment, the oligonucleotide binding agents are arranged in a three dimensional manner, which faciliates binding of the respective labelling agents.

Other preferred binding agents are shown in the following table, which is not to be understood as limiting the scope of the present invention. Binding agent 1 can for example act as the complementary binding unit according to the invention, which binds, or forms part of, the labelling agent, and binding agent 2 can be bound to, or act as, the anchoring unit, or vice versa.

In a preferred embodiment, the anchoring unit can consist essentially of either binding agent 1 or 2. In this case, it is directly bound to the scaffold, and has a free binding moiety for the complementary binding unit which binds, or forms part of, the labelling agent.

Many of the biocompatible binding agents shown provide as well a controlled binding and cleaving, as discussed above for oligonucleotides, and are thus useful for *in vivo* applications (see below).

**Table 4**

| agent 1 | agent 2 (complementary) |
|---|---|
| oligonucleotide | complementary oligonucleotide |
| molecular tag | complementary moiety |
| antibody | antigen |
| ankyrin | complementary moiety |
| lectin | sugar, glycoproteins, glycolipds |
| biotin | streptavidin, avidin |
| collagen binding proteins | collagen |
| ligand | tissue specific receptor |
| tissue specific ligand | receptor |
| magnetic beads | magnetic beads of complementary polarity |
| charged group (*e.g.,* "+") | complementarily charged group (*e.g.,* "-") |
| zwitterions | complementary zwitterion |
| hydrophilic group | hydrophilic group |
| hydrophobic group | hydrophobic group |
| bifunctional group | complementary bifunctional group |

The term "zwitterion", as used herein, refers to a molecule has at least one pair consisting of a positively charged group and a negatively charged group. It is crucial to the invention that in this case a complementary zwitterion exists, so that both zwitterions can act as complementary binding agents.

The term "bifunctional group", as used herein, refers to a group which has at least one pair consisting of a hydrophilic and a hydrophobic portion, or subdomain. It is crucial to the invention that in this case a complementary bifunctional group exists, so that both bifunctional groups can act as complementary binding agents.

The term "molecular tag" (sometimes also termed "affinity tag"), as used herein, refers to molecules which are, for example, being used for the purification of proteins. Examples for these tags are shown, in a non-limiting fashion, in table 5.

**Table 5**

| Molecular tag | Complementary moeity |
|---|---|
| immobilized metal ions, like Ni-NTA | His-tag (Hexahistidine) |
| his-tag (Hexahistidine) | immobilized metal ions, like Ni-NTA |
| chitin binding protein (CBP) | chitin |
| maltose binding protein (MBP) | amylose |
| rProtein L | kappa light chain of immunoglobulins (Cκ domain) |
| Flag-Tag (DYKDDDDK) | antibody |
| Strep-tag | biotin |
| Hemagglutinin-tag (HA) | immunoglobulin (*e.g.*, ABIN130391) |
| myc-tag | immunoglobulin 9E10 |
| GST (Glutathion-S-Transferase) | glutathione |

(continued)

| Molecular tag | Complementary moeity |
|---|---|
| V5-tag | immunoglobulin (*e.g* ab9113) |
| BCCP tag (Biotin Carboxyl Carrier protein) | avidin |
| Calmodulin-tag | calcium |
| GFP-tag (green fluorescent protein) | immunoglobulin |

It is another striking advantage of the bio-orthogonal binding mechanisms discussed above that they reduce the risk of impairing colonization of the scaffold with cells, or division of cells which have just colonized the scaffold. This is basically due to the fact that the above discussed bio-orthogonal binding mechanisms, or binding agents (like azide groups or oligonucleitoides) are less likely to have detrimental effects on cells than most of the labelling agents discussed above.

By means of illustration, not by means of limitation, other reaction types for binding a labelling agent to an anchoring unit are shown in the following table, which is not to be understood as being part of the present invention in its entirety.

**Table 6**

| Reaction type | example |
|---|---|
| cycloaddition | Huisgen 1,3-dipolar cycloaddition Diels-Alder reaction |
| nucleophilic substitution | small strained rings like epoxy and aziridine compounds |
| carbonyl-chemistry-like formation of urease | |
| addition reactions to carbon-carbon double bonds | dihydroxylation |

In yet another preferred embodiment of the present invention, the polymeric scaffold materials and/or scaffolds are produced by at least one method selected from the group consisting of

a) electrospinning,
b) rapid prototyping,
c) knitting, and/or
d) phase separation

Electrospinning is a method for generating ultrathin fibers from materials such as polymers, composites, and others. Nanofibers of both solid and hollow interiors ("nanotubes") can be formed as well. The thin fibers are produced by uniaxial stretching of a viscoelastic jet derived from a polymer solution or melt by applying high voltages. The fibers are deposited on a flat surface and lead to a complex meshwork which afterwards can be shaped. The electrospinning process for making scaffolds is known in the art as described by Van Lieshout *et al.* (2006).

The term "Rapid prototyping" as used herein, refers to methods for the construction of physical objects using solid freeform fabrication, *i.e.,* without a mould. Rapid prototyping takes virtual designs from computer aided design (CAD) or animation modeling software, transforms them into thin, virtual, horizontal cross-sections and then creates each cross-section in physical space, one after the next until the model is finished. Table 7 gives a non-limiting overview of some rapid prototyping methods which can be used in the context of the present invention.

**Table 7**

| Prototyping Technologies | Base Materials (examples) |
|---|---|
| Selective laser sintering (SLS) | thermoplastics, metals powders |
| Fused Deposition Modeling (FDM) | thermoplastics, eutectic metals. |
| Stereolithography (SLA), or three dimensional lithography | photopolymerizable polymers |
| Electron Beam Melting (EBM) | titanium alloys |
| 3D Printing (3DP) | various |

(continued)

| Prototyping Technologies | Base Materials (examples) |
|---|---|
| Solid ground curing (SGC) | photopolymerizable polymers |

Knitting is a method well known in the art. In scaffold production, it allows the production of an open structure that is mechanically reliable. An advantage of knitting is the complex geometries that can be produced, *e.g.,* branched prostheses used for aortic arch replacements Among the materials that have been used for knitting are Dacron, but also carbon fibers as well as polymers like polycaprolactone. The knitting process for making scaffolds is as well described by Van Lieshout *et al.* (2006).

Phase separation comprises different approaches, them being immersion precipitation, solid-liquid phase separation, liquid-liquid phase separation, polymerization-induced phase separation and particularly thermally induced phase separation ("TIPS"). The lattter is a method that requires the use of a solvent with a low melting point that is easy to sublime. This can for example be done with dioxane as a solvent for polylactic acid. Phase separation is then induced by addition of a small quantity of water, which leads to the formation of a polymer-rich phase and a polymer-poor phase. The mixture is then cooled below the solvent melting point, and vacuum-dried, to sublime the solvent in order to obtain a porous scaffold.

Other methods for the production of scaffolds which fall under the scope of the present invention comprise at least one selected from the group consisting of

- gas foaming with injected gas, $CaCO_3$ or $NH_4HCO_3$
- sintering of microspheres
- Super critical fluid technology
- Particulate leaching
- Emulsification
- Freeze drying
- Solvent casting
- Extrusion
- Production of Nonwovens
- Weaving
- Fibre bonding
- Membrane lamination
- Hydrocarbon templating
- Solid Freeform Fabrication techniques
- Mould casting, and/or
- Microrobotics/micromachining

In another preferred embodiment, the scaffold material is a biodegradeable material. Such biodegradeable material can be selected from, among others,

collagen I, collagen II, collagen III, collagen IV, collagen V, collagen VI, collagen VII, collagen VIII, collagen IX, collagen X, elastin, poly(lactide-co-glycolides) (PLGA), polycarpolactone (PCL), polylactic acid (PLA), polyglycolic acid (PGA), tissue culture plastic (TCP), *poly*(propylene fumarate (PPF), poly(ethylene glycol) terephthalate (PEGT), poly(butylene terephthalate), Peptide Hydrogels (*e.g.,* PuraMatrix™), polysaccharidic materials, particularly chitosan or glycosaminoglycans (GAGs), hyaluronic acid, particularly in combination with cross linking agents (*e.g.,* glutaraldehyde, paraforaldehyde, or water soluble carbodiimide), or mixtures, copolymers or modifications (see example 1) thereof.

The person skilled in the art has a good understanding of the respective polymerization reactions, and the respective monomers used.

In another preferred embodiment of the present invention it is provided that the anchoring unit is added to the reaction mixture prior to polymerization.

In this embodiment, the anchoring unit is added, *e.g.,* to the monomer mixture prior to polymerization. In case of PLGA, which is a copolymer of the cyclic dimers (1,4-dioxane-2,5-diones) of glycolic acid and lactic acid, and synthesized by means of random ring-opening co-polymerization, the anchoring unit is thus added to the said cyclic dimmer. This approach provides for an even distribution of the anchoring unit in the later scaffold, and thus a uniform labelling of the scaffold, but is only available if the presence of the anchoring unit does not interfere with the polymerization process.

In an alternative embodiment it is provided that the anchoring unit is attached to the polymer from which the scaffold is formed.

In this embodiment, the anchoring unit is added, *e.g.,* to the polymer prior to formation of the scaffold, *e.g.,* by electrospinning and/or rapid prototyping. This again yields for an even distribution of the anchoring unit in the later scaffold.

In yet another alternative embodiment it is provided that the anchoring unit is attached after the forming of the scaffold.

In this embodiment, the anchoring unit is mainly attached to the surface of the scaffold. This makes sense for *in vivo* labelling applications (see below), as here the binding of the labelling agent can only take place on the surface of the scaffold.

The attachment of the anchoring unit to the scaffold is, in a preferred embodiment, a covalent binding step.

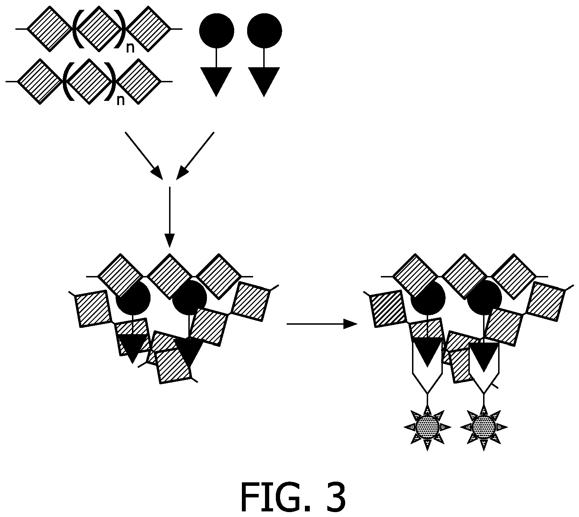

In yet another preferred embodiment, the attachment of the anchoring unit to the scaffold is a non-covalent binding step, as for example shown in Fig. 3.

In a particularly preferred embodiment, the method according to the invention is characterized in that the labelling agent is bound to the anchoring unit *in vivo.*

The term *"in vivo",* as used herein, shall refer to a binding process which takes place once the scaffold is implanted in a patient. It can be used interchangeably with the term *"in situ".*

This is a particularly advantageous approach, as it allows to expose the body of a patient to a labelling agent, which is in some cases a potentially allergenic, or even toxic, agent, for a period as short as possible, *e.g.,* only directly before the imaging process.

In order to monitor the continuous bio-degradation of the scaffold and to assess the structural and mechanical properties during the degradation of an implanted scaffold, one may thus provide the said scaffold, prior to implantation, with at least one anchoring unit according to the invention.

It is particularly preferred that, in the *in vivo* approach, the labelling agent is released again after the imaging experiment. Some of the binding agents shown above allow for such release.

When need for a scaffold check arises, one may then administer a labelling agent to the patient, which, once in the blood flow, binds to the anchoring units, allowing thus a proper imaging of the complete scaffold. After the medical examination, the binding can be cleaved and the labelling agent is released, and will be excreted with the urine, for example.

This approach is particularly useful in case a scaffold check examination needs to be done only after a longer period of time, as some biodegradation processes take months, or even years. In many cases, labelling agents, like gadolinium (Gd)-based agents, have poor biocompatiblity, and it is not desirable to have these agents in the body for an extended period of time, as Gd could be released from the complexes and lead to health problems if residing too long in the body. However, methods according to the prior art provide that the labellling agent is added to the scaffold, or the scaffold material, prior to implantation.

In contrast thereto, the *in vivo* use allows adding even after an extended period of time. This means that the labelling agent can be added, *e.g.,* one year after the scaffold was implanted into the body. This can be advantageous as during this extended time the imaging label can be degraded, or released, if introduced prior to implantation, thus degrading the labelling function over time.

For the *in vivo* use, a controlled binding and cleaving, as mentioned above, has several advantages, as it allows the time controlled binding, and removal, of a labelling agent

Furthermore this approach faciliates the use of radionuclides as labelling agents (see table 1), which are necessary for some imaging modalities, such as PET, SPECT, scintigraphy or other nuclear medicine tomographic imaging techniques (see table 1)

However, in another preferred embodiment, the labelling agent is bound to the anchoring unit *in vitro.* The term *"in vitro",* as used herein, shall refer to a binding process which takes place prior to implantation of the scaffold. The advantage of the *in vitro* binding is that harsher conditions for the chemical coupling reaction can be applied, resulting in a more reliable binding. Furthermore, a greater number of binding reactions is available for the *in vitro* approach.

A scaffold is, in a preferred embodiment, obtainable by a method according to the invention.

Said scaffold is, in another preferred embodiment being used for the production of at least one tissue and/or organ selected from the group consisting of

    a) artificial heart valves,
    b) vascular grafts,
    c) skin,
    d) nervous tissue,
    e) organs,
    f) bladder,
    g) blood vessels,
    h) cartilage tissue, and/or
    i) bone tissue.

Furthermore, the use of such scaffold for the manufacture of a tissue and/or organ is provided, as well as a tissue and/or organ comprising such scaffold. Said tissue and/or organ is preferably selected from at least one of the group consisting of

a) artificial heart valves,
b) vascular grafts,
c) skin,
d) nervous tissue,
e) organs,
f) bladder,
g) blood vessels,
h) cartilage tissue, and/or
i) bone tissue.

The invention comprises, in another embodiment, a method of labelling a scaffold material and/or a scaffold for tissue and/or organ engineering according to the invention, said method comprising the step of binding at least one labelling agent to the anchoring unit.

The at least one labelling agent is bound to the anchoring unit by means of a bio-orthogonal chemical reaction.

It is furthermore preferred that at least one labelling agent is bound to the anchoring unit *in vivo.* In another preferred embodiment, the method comprises the step of releasing at least one labelling agent from the anchoring unit *in vivo.*

The following table gives a non-limiting overview of some scaffold materials, anchoring units, complementary binding units and labelling agents according to the present invention. It is to be noted that, in most cases, anchoring unit and complementary binding unit can be used interchangeably. Furthermore, it is to be noted that materials mentioned in column 1, 2 - 3 and 4 can be used interchangeably, e.g., the scaffold material of line 3 can be used with labelling agent of line 8, and connected to one another by the anchoring unit and its complementary binding unit of line 1, and so forth.

**Table 8**

| Scaffold material | Anchoring unit | Complementary binding unit (if nessesary) | Labelling agent |
|---|---|---|---|
| Collagen I - X | Oligonucleotide | complementary oligonucleotide | Barium based agents, *e.g.,* barium sulphate based agents |
| Elastin | molecular tag (table 5) | complementary moiety | iodine based agents |
| poly(lactide-co-glycolides) (PLGA), polycarpolacto ne (PCL), polylactic acid (PLA), polyglycolic acid (PGA) | biocompatible binding agent (table 4) | complementary moiety | Gadolinium-complexes |
| tissue culture plastic (TCP), *poly* (propylene fumarate (PPF), poly (ethylene glycol) terephthalate (PEGT), poly(butylene terephthalate) | Azide residue  "Staudinger ligation" | phosphine or phosphate residue | non-ionic Iodine based agents |
| Peptide Hydrogels (*e.g.,* PuraMatrix™) | Azide residue  "Click reaction" | organic carbon with at least one C=C triple binding, presence of copper as catalyst | Lanthanaide ions and complexes, *e.g.,* Gadolinium-complexes, like Gd-DTPA-BMA |
| polysaccharidic materials, particularly chitosan or glycosaminoglycans (GAGs) | Azide residue  "Strain promoted cycloaddition" | cylic carbon with at least one C=C triple binding | PEG-coated iron oxide |

(continued)

| Scaffold material | Anchoring unit | Complementary binding unit (if nessesary) | Labelling agent |
| --- | --- | --- | --- |
| hyaluronic acid, particularly in combination with cross linking agents (*e.g.*, glutaraldehyde , or water soluble carbodiimide) | | | SPIO/Iron oxide nano particles (silicon coated, superparamagnetic) |
| | | | manganese based agents (Mangan-DPDP) |
| | | | manganese oxide nano particles |
| | | | perfluorooctyl Bromide |
| | | | bariumsulfate-suspensions |
| | | | Iron Oxide |
| | | | $^{19}$F containing materials, *e.g.,* perfluorooctyl bromide (PFOB) |
| | | | gas filled microbubbles |
| | | | $^{11}$Carbon, $^{13}$Nitrogen, $^{15}$Oxygen, $^{18}$Fluorine, $^{19}$Fluorine, all (optionally) organically bound |
| | | | $^{99}$Technetium, $^{123}$Iodine and $^{131}$Iodine, $^{201}$Thallium, $^{67}$Gallium, $^{18}$Fluorine, Fluorodeoxyglucose, $^{111}$Indium |

DEFINITIONS

The term "tissue engineering", as used herein, refers to an interdisciplinary field that applies the principles of engineering and life sciences toward the development of biological substitutes that restore, maintain, or improve tissue function or a whole organ. It comprises the use of a combination of cells, engineering and materials methods, and suitable biochemical and physio-chemical factors, to improve or replace biological functions, particularly tissues and/or organs.
This includes the repair or replacement of portions of, or whole, tissues and/or organs (*i.e.*, bone, cartilage, blood vessels, bladder, etc.). sometimes resulting in artificial organs and/or tissues, like an artificial pancreas, or a bioartificial liver. Tissue engineering requires, in most cases, a scaffold and living cells to colonize the former.
The term "scaffold", as used herein, relates to a three dimensional matrix on which cells are grown. These matrices are often critical, both *ex vivo* as well as *in vivo,* to recapitulating the *in vivo* milieu and allowing cells to influence their own microenvironments. Scaffolds usually serve at least one of the following purposes:

- Allow cell attachment and migration
- Deliver and retain cells and biochemical factors
- Enable diffusion of vital cell nutrients and expressed products
- Exert certain mechanical and biological influences to modify the behaviour of the cell phase

To achieve the goal of tissue reconstruction, scaffolds must meet some specific requirements. A high porosity and an adequate pore size are necessary to facilitate cell seeding and diffusion throughout the whole structure of both cells and nutrients. Biodegradability is often an essential factor in case the scaffolds are supposed to be absorbed by the surrounding tissues over time without the necessity of a surgical removal. The rate at which degradation occurs has to coincide as much as possible with the rate of tissue formation. This means that while cells are fabricating their own natural matrix structure around themselves, the scaffold provides structural integrity within the body, and eventually it will break down leaving the so called neotissue, *i.e.*, newly formed tissue which will take over the mechanical load.

Cells as used for tissue engineering comprise, among others, fibroblasts and/or keratocytes (for skin replacement or repair), chondrocytes (for cartilage replacement or repair), stem cells (large variety of potential tissues to be replaced, or repaired), pluripotent cells (large variety of potential tissues to be replaced, or repaired), cardiac stem cells (for the repair or replacement of cardiac tissue), endothelial stem cells (for the repair or replacement of vascular tissue), valve stem cells (for the repair or replacement of heart valves), and so forth.

The cells used comprise, extended telomeres, in order to increase their dividing potential and/or lifetime, which is restricted, in non-modified cells, by the so-called Hayflick limit.

Particularly preferred, the cells used are autologous cells, *i.e.*, cells which are genetically compatible with the recipient of the tissue or organ produced therewith. This is basically the case if the cells are derived from the same subject to which the cells are applied (*i.e.*, donor and recipient are the same person), or in case donor and recipient are close relatives.

The terms "complementary nucleic acid" and "complementary oligonucleotide" refer to nucleic acids, polynucleotides and/or oligonucleotides which have base sequence comprising any of the bases cytosine (C), guanine (G), adenine (A), thymine (T) and uracil (U), or to Hypoxanthine, Xanthine, 7-Methylguanine, 5,6-Dihydrouracil, 5-Methylcytosine, isoguanine and isocytosine, that is capable of hybridizing to another nucleic acid, polynucleotide and/or oligonucleotide according to the Watson-Crick base pairing mechanism.

The terms "antibody" and "monoclonal antibody" refer to immunoglobulin molecules exhibiting a binding affinity towards a given antigen, and which are either produced by immunized mammals, or by recombinant microorganisms.

Lectins are sugar-binding proteins which are highly specific for their sugar moieties. They typically play a role in biological recognition phenomena involving cells and proteins. For example, some bacteria use lectins to attach themselves to the cells of the host organism during infection.

Ankyrin repeats are derived from natural ankyrin repeat proteins which are used in nature as versatile binding proteins with diverse functions such as cell signalling, kinase inhibition or receptor binding just to name a few. These ankyrin repeats are for example described in EP1332209.

Streptavidin is a 53 Kd protein purified from the bacterium Streptomyces avidinii, which exhibits strong affinity for the vitamin biotin; the dissociation constant (Kd) of the biotin-streptavidin complex is on the order of ~10-15 mol/L. Avidin is a similar protein which has as well a strong affinity to biotin.

The term "labelling agent", as used herein, refers to an agent, *i.e.,* a molecule, which can be made visible by means of an imaging apparatus, like an X-ray, a computer tomograph (CT, particularly spectral CT, a Magnetic Resonance Imager (MRI), a sonograph, a positron emission tomograph (PET) and/or a scintigraph (see table 1). The visualization is particularly useful when an embodiment labelled with said labelling agent is implanted into the human body. In this case, on would speak of *in situ* visualization, or *in vivo* visualization. Frequently, the said labelling agents are also termed "contrasting agents".

## DISCUSSION OF THE FIGURES

The following figures illustrate schematically the essential aspects of the invention.

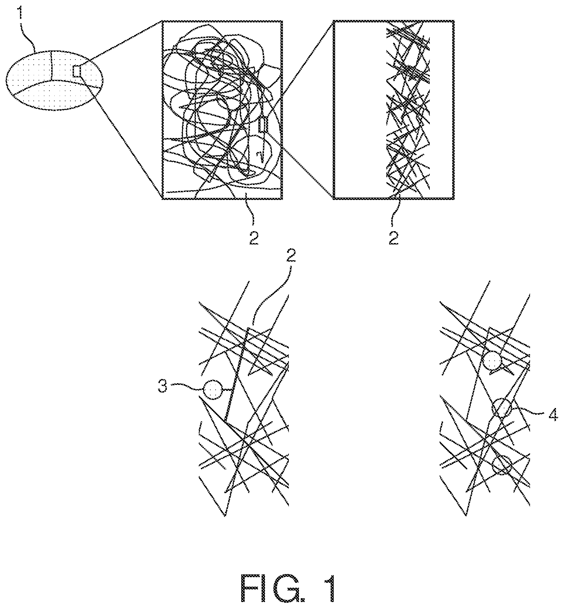

Fig. 1 shows, in an exemplary fashion, a scaffold 1 for an artificial heart valve. The scaffold is made by electrospinning of a polymer, a mixture of different non-modified or modified polymers, or a mix of a polymer and other materials/entities. This leads to a material comprising long fibers 2, to which anchoring units 3, 4 according to the invention are attached.

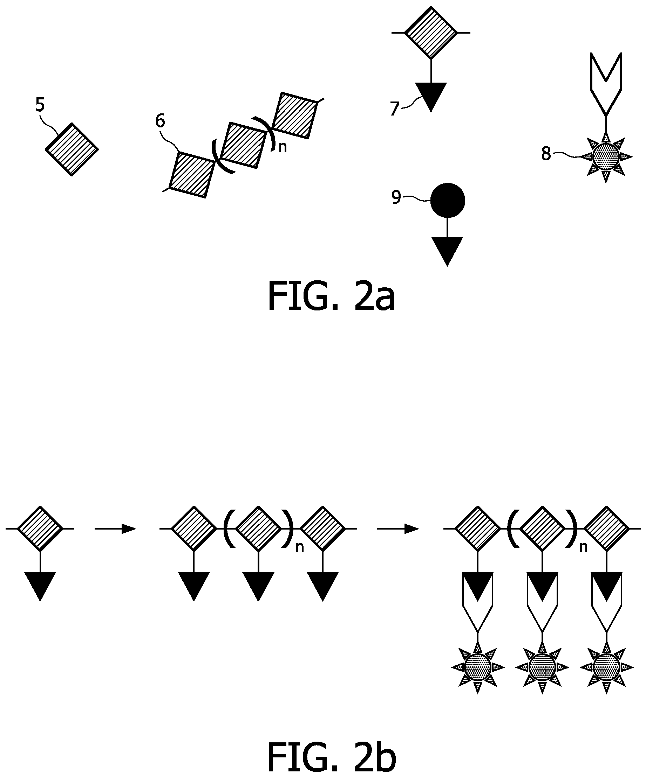

Fig. 2a shows some elements of the invention, namely monomers 5, a polymer 6, an anchoring unit 7 covalently bound to a monomer 5, a labelling agent 8, and another anchoring unit 9 not covalently bound to a monomer.

The labelling agent 8 can be bound to, or form part of, an entity which allows binding to the anchoring unit 7 or 9. This entity is also called "complementary binding unit".

The anchoring unit 9 is bound to a particle which can be incorporated, non-covalently, in a scaffold during the electrospinning process.

Fig. 2b shows a preferred embodiment of the invention in which the attachment of the anchoring units takes place prior to polymerization of the monomers. The polymers thus produced can then undergo electrospinning. Later on, labelling agents can be bound to the anchoring units, particularly *in vivo.*

Labelling agents to be used for MRI can be $^{19}$F-containing materials, chemical shift agents to shift proton signal of protons in the scaffold or lanthanide ions, e.g., gadolinium Gd-containing complexes. Other agents that can be used for MRI are of course also possible. Furthermore, labelling agents to be used for other imaging modalities (CT, X-ray) can be used as well. Gd-complexes can also be used for spectral CT, which is more sensitive than conventional CT (see table 1).

Fig. 3 shows a process in which anchoring units not covalently bound to a monomer are mixed with polymerized scaffold matter, and do then undergo a co-electrospinning, in such way that the anchoring agents are incorporated in the fibers thus produced. Later on, labelling agents can be bound to the anchoring units, particularly *in vivo.*

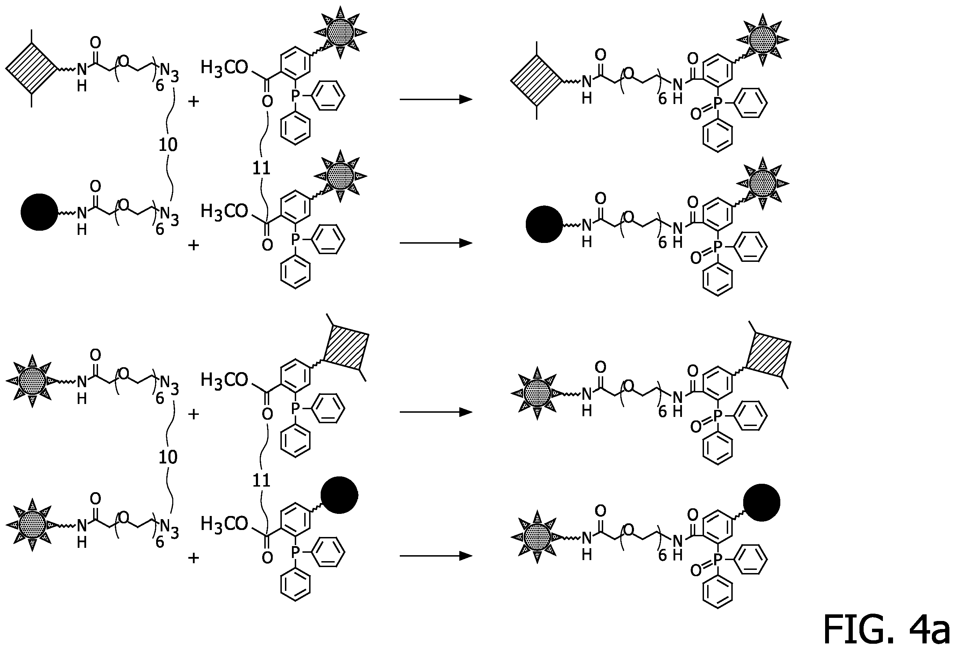

Fig. 4a shows, as an example for the bio-orthogonal binding of a labelling agent to an anchoring unit, the so-called Staudinger ligation. The monomer comprises a chemical modification which acts as an anchoring unit according to the invention, namely an azide ($N_3$-group) 10, which can click to a phosphine group 11 which carries the labelling agent.

Alternatively, in a reverse fashion, the monomer can also be coupled to the phosphine group (11), which does in this case act as the anchoring unit according to the invention, and then the labelling agent is attached, by its azide group (10), to the anchoring unit.

The anchoring unit and the complementary binding unit of the labelling agent can as well be complementary strands of oligonucleotides, which can form stable non-covalent bonds *in vivo* and are able to target the label specifically to the site where it is needed.

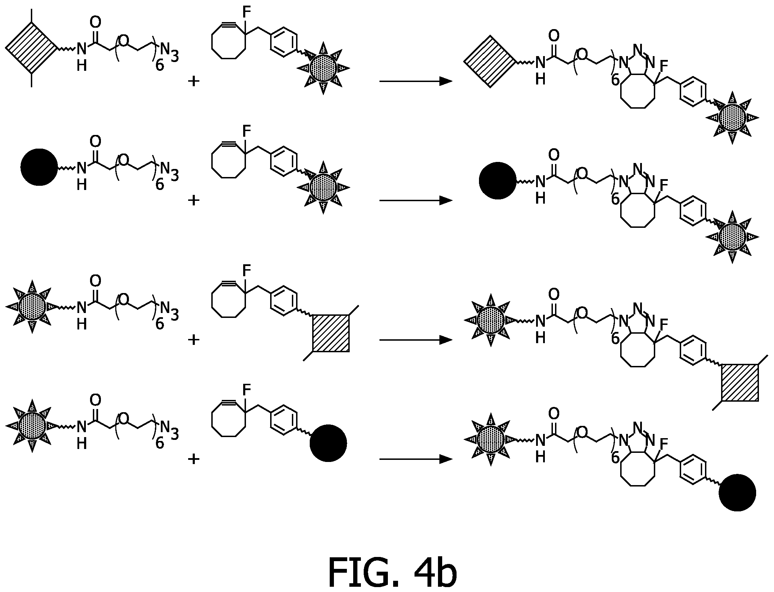

Fig. 4b shows, as another example for the bio-orthogonal binding of a labelling agent to an anchoring unit, the so-called click-reaction, in which the modified monomer clicks to a strain-stressed alkyne.

Fig. 5a shows a single-stranded oligonucleotide bound to an anchoring unit, and a complementary single-stranded oligonucleotide bound to a labelling agent. Both can hybridize with each other, particularly in an *in vivo* situation, thus binding a labelling agent to a scaffold just in time (see above). Under certain circumstances, the hybridization can later be cleaved again, *e.g.,* by application of locally focused heat or by restriction enzymes.

Fig. 5b shows two double-stranded oligonucleotides with sticky ends, which can be used as binding agents according to the invention. The overhangs are complementary to one another, thus allowing a binding by hybridization. One of the shown oligonucleotides may be coupled to an anchoring unit, or even form part of it. The said complementary oligonucleotides allow, for example, a controlled binding and cleaving of the labelling agent to the scaffold. These features make the said complementary oligonucleotides particularly useful for *in vivo* binding, as discussed below.

Again, cleaving can take place by application of heat, or a restriction enzyme. In the latter case, it is preferred that the cleaving site of the restriction enzyme corresponds to the sequences of the sticky ends.

Fig. 6 shows different examples of other nucleotides serving as binding agents according to tzhe invention. In Fig 6a, an oligonucleotide with 40 residues (40-mer), is shown together with two complementary oligonucleotides with 10 residues each (10-mers), each being coupled to a labelling agent.

In Fig. 6b, the oligonucleotide has two sections being complementary to two oligonucleotides which carry a labelling agent each, the two sections being separated from one another by an oligomer with a length of n residues.

In Fig. 6c, the oligonucleotide has three sections being complementary to three oligonucleotides which carry a labelling agent each, thus allowing the use of three different labelling agents

In Fig. 6d, an oligonucleotide is shown which comprises a spacer which seperates two sequences from one another, thus allowing for a spatially separated hybridization of two complementary oligonucleotides which carry a labelling agent each.

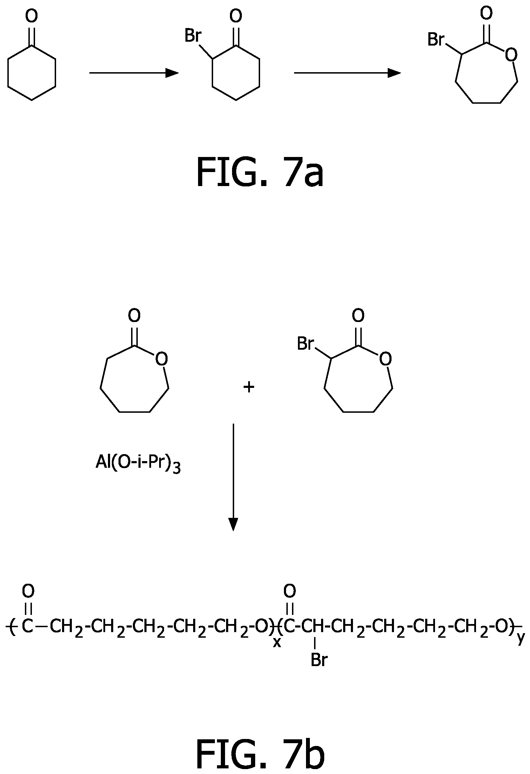

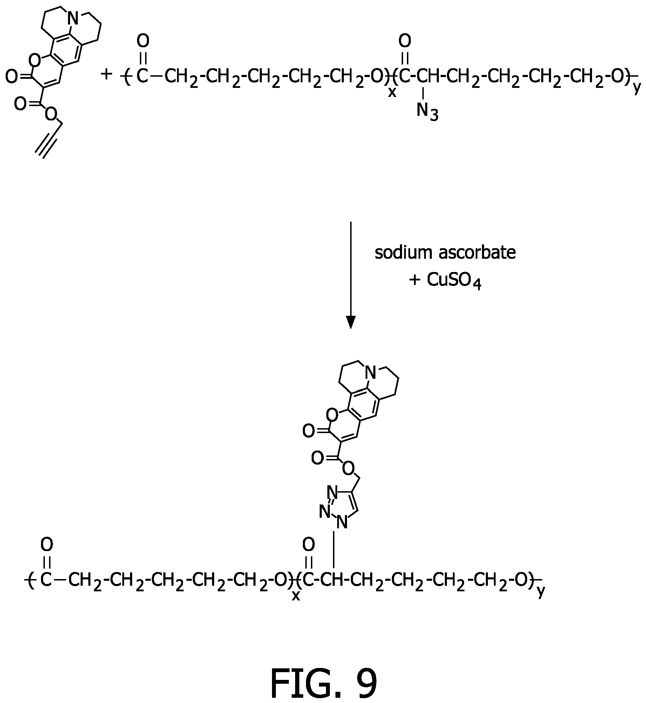

Figs. 7 - 9 show the steps of labelling a scaffold with click chemistry comprising a covalently bound anchoring unit. These Figures are discussed in connection with example 1.

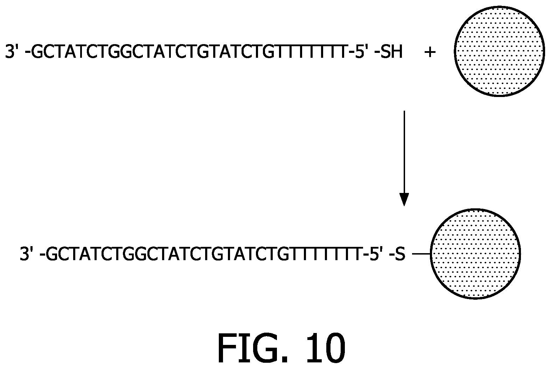

Figs. 10 - 12 show the steps of labelling a scaffold by means of Gd-labelled oligonucleotides. These Figures are discussed in connection with example 3.

Fig. 13 shows several oligonucleotide binding agents linked to a spherical bead incorporated into the scaffold material. In this embodiment, the oligonucleotide binding agents are arranged in a three dimensional manner (*e.g.,* in a tetraedric shape, while, in Fig. 13, this is projected in a two dimensional manner). The spherical bead is, in this example, a Gold particle, as described in example 3. The three dimensional arrangement has several advantages. First, any steric interactions between the different labelling agents which bind to the oligonucleotide binding agents are reduced to a minimum. Furthermore, the arrangement provides the option to use different oligonucleotide binding agents (*i.e.*, which differ from one another by their sequences, as shown in Fig. 13), in order to allow the use of different labelling agents which in turn might be useful for different imaging devices.

Another advantage is that such arrangement improves the binding properties in case the spherical bead is incorporated in a scaffold material. In such way the likelihood is increased that at least one binding agent peeks put of the scaffold material and is thus available for binding to a labelling agent. In this case, the different oligonucleotides may have the same sequence preferably.

EXAMPLES

Example 1: Labelling of a scaffold with click chemistry comprising a covalently bound anchoring unit.

A copolymer of ε-caprolactone and α-bromo-ε-caprolactone (or a pure α-bromo-ε-caprolactone) is being produced, which serves as a polymer for formation of the scaffold. This process is described in examples 1.1. - 1.3. Then the Br-groups of the copolymer are being substituted by azide ($N_3$)-groups, the latter being the anchoring units of the present invention. This process is described in example 1.4. The copolymer can undergo a scaffold formation process, *e.g.,* by electrospinning, prior or after the substitution process. Labelling agents are being bound to the anchoring units by means of a staudinger reaction.

### 1.1. Synthesis of $\alpha$-bromocyclohexanone

$\alpha$-bromocyclohexanone is synthesized according to the following procedure: To a stirred mixture of 30 g (0.306 mol) of cyclohexanone and 200 mL of distilled water, 49 g (0.306 mol) of bromine is added dropwise over a period of 5 h, during which the temperature is maintained between 25 and 30 °C by external cooling.

When addition is completed, stirring is continued until the reaction mixture is colorless (about 1 h). The heavy organic layer is separated from the aqueous layer and dried over anhydrous $MgSO_4$. Pure $\alpha$-bromocyclohexanone (37 g, 69% yield) is then obtained by distillation. See Fig. 7a for a reaction scheme.

### 1.2. Synthesis of $\alpha$-bromocaprolactone ($\alpha$BrCL)

31 grams of 3-chloroperoxybenzoic acid (mCPBA, 0.135 mol) are added to a solution of 21.8 g (0.123 mol) of $\alpha$-bromocyclohexanone in 200 mL of dichloromethane. After stirring at room temperature for 8 h, the reaction flask is placed in a refrigerator in order to precipitate 3-chlorobenzoic acid generated in the reaction. The solution is then filtered and washed with saturated solution of $Na_2S_2O_3$ three times, with a solution of $NaHCO_3$ three times, and finally with distilled water until neutral pH. The organic phase is dried with anhydrous $MgSO_4$ overnight. After $MgSO_4$ is filtered off, the solvent is removed by rotary evaporation. The crude product is dissolved in a mixture of hexane and ethyl acetate (10/3, volume ratio) and passed through a silica gel column prepared with the same solvent, collecting the second fraction. The solvent is removed by rotary evaporation, and the white solid is dried under vacuum overnight at room temperature. Yield: 12.5 g (53%). Melting point: 34.5 8C. See Fig. 7a for a reaction scheme.

### 1.3. Copolymerization of $\varepsilon$-caprolactone ($\varepsilon$CL) and $\alpha$BrCL via ring opening polymerization (ROP)

Random ring opening polymerization is carried out at 25 °C in toluene. To a 50 mL polymerization flask which is degassed by five vacuum-argon cycles, 15 mL of toluene, 0.628 g (3.25 mol) of $\alpha$BrCL in toluene, 5.507 g (48.31 mmol) of $\varepsilon$CL, and 0.307 g of aluminum isopropoxide (Al(O-i-Pr)$_3$, 1.5 mmol) in toluene are successively added through a rubber septum with a syringe. After polymerization for 3 h, an excess of 1 N HCl is added, and the is recovered by precipitation in cold methanol. See Fig. 7b for a reaction scheme.

### 1.4. Substitution of Br-groups

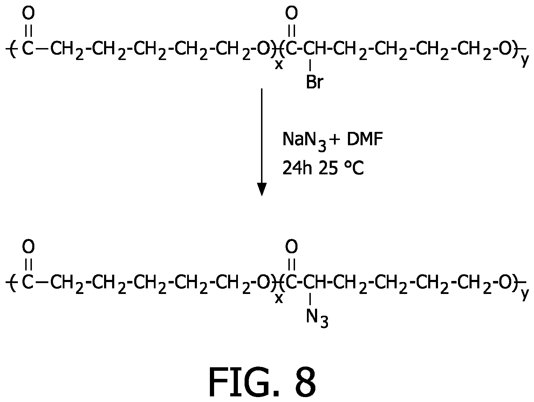

The copolymer thus produced is immersed in a saturated solution of sodium azide in 2,5-dimethylfurane (DMF) for 24 h at room temperature. The substrates are then rinsed with DMF, sonicated 3 min in ethanol and 3 min in water, and dried in a stream of air. This process leads to a substitution of Br-groups by azide ($N_3$)-groups, the latter being the anchoring units of the present invention. See Fig. 8 for a reaction scheme.

### 1.5. Binding of a labelling agent by means of click chemistry

A coumarin dye is being used as an exemplary labelling agent. A click reaction between the azide groups of the copolymer and the propyne groups of a coumarin 343 derivative (10-oxo-2,3,5,6-tetrahydro-1H,4H,10H-11-oxa-3aaza-benzo[de]anthracene-9-carboxylic acid prop-2-ynyl ester) is carried out by immersing the azide-functionalized substrate for 24 h in a solution of the coumarin 343 derived (5 mg, 0.015 mmol) dissolved in 10 ml ethanol at room temperature.

$CuSO_4 \times 5\ H_2O$ (0.19 mg, $7.73 \times 10$-4 mmol, 5 mol%) and sodium ascorbate (0.31 mg, $1.55 \times 10$-3 mmol, 10 mol%), each dissolved in 1 ml of water, are added as catalysts. Subsequently, the substrates are sonicated in ethanol for 3 min and dried in a stream of air.

The said reaction has a particular advatage that it can be carried out *in vivo*.

Furthermore, the reaction can be carried out with all labelling agents that can be provided with a terminal alkyne group (-C=CH, or $-\equiv$).

### Example 2: labelling of an oligonucleotide binding agent with $^{18}$F

An oligonucleotide as shown in Fig. 5 is labelled at its 5'-end with $^{18}$F acccording to the method of Kuhnast 2003. $^{18}$F is preferably used in positron emission tomography (PET) and scintigraphy (see table 1). A complementary oligonucleotide is anchored to a scaffold material by means according to the art, thus serving as an anchoring unit according to the invention. Methods to bind an oligonucleotide to a silicate surface or to a polymer surface are for example known from the literature related to the manufacture of biochips.

Example 3: Labelling of a scaffold by means of Gd-labelled oligonucleotides

3.1. Synthesis of the DNA-particle-component

Au (Gold) particles can be prepared as described in literature (Grabar *et al.* (1995)). These particles are easily modified with oligonucleotides, which are functionalized with alkane thiols at one of their termini, *e.g.,* the 5'terminus. Here, a solution of 1.5 ml (17 nM) Au colloids (13 nm Ø) is treated for 24 h with 460 $\mu$l (3.75 $\mu$M) SH-5'-Oligonucleotides-3' of the following kind (sequence is randomly selected here, *i.e.,* any other sequence will do as well):
3'-GCTATCTGGCTATCTGTATCTGTTTTTTTT-5'-SH

in order to provide DNA-Gold- components In such way, an anchoring unit comprising an oligonucleotide as a binding agent is obtained. See Fig. 10 for an illustration of said process.

3.2. Synthesis of the DNA-Gd-label component

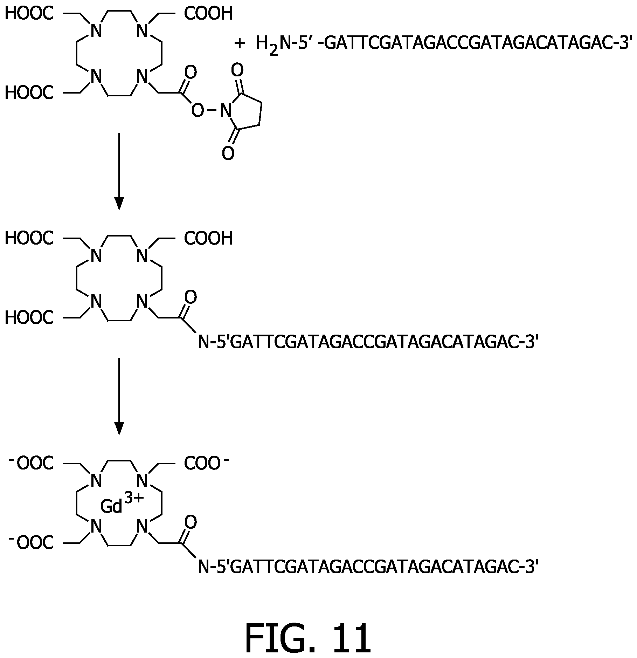

1 $\mu$mol amine-modified oligonucleotide of the following kind (part of sequence is complementary to the above sequence):
$H_2N$-5'-GATTCGATAGACCGATAGACATAGAC-3'
is dissolved in 1000 $\mu$l PBS. To this solution 6 $\mu$mol DOTA-NHS-ester (DOTA-NHS-ester = 1,4,7,10-Tetraazacyclododecane-1,4,7,10-tetraacetic acid mono(N-hydroxy-succinimide ester)) dissolved in 1000 $\mu$l PBS is added. The latter carries an amine-reactive succinimidyl ester moiety.
The mixture is then agitated at room temperature in order to carry out a subsitution reaction.
The product is dialyzed against pure $H_2O$ (cut-off of 6-8 kDa). Then, 1 $\mu$mol $GdCl_3 \cdot 6H_2O$ (stock solution of 20 mg/ml) is added. The pH is kept constant between 5.0 and 5.5 (by adding IN HCl or IN NaOH) over night. Then, 1 $\mu$mol EDTA is added in order to chelate excess $Gd^{3+}$. After stirring for 30 min, the milky solution can be purified with a Sephadex G-25 column to remove the EDTA-$Gd^{3+}$ and other unreacted low molecular weight compound from the DNA-DOTA-Gd complex. DOTA-NHS-ester is commercially available, *e.g.,* from Macrocyclics, Dallas, Texas, or CheMatech, Dijon, France).
In such way, a Gd-based labelling agent comprising an oligonucleotide as a complementary binding agent is obtained. See Fig. 11 for an illustration of said process.

3.3. Use in scaffolds for tissue engineering

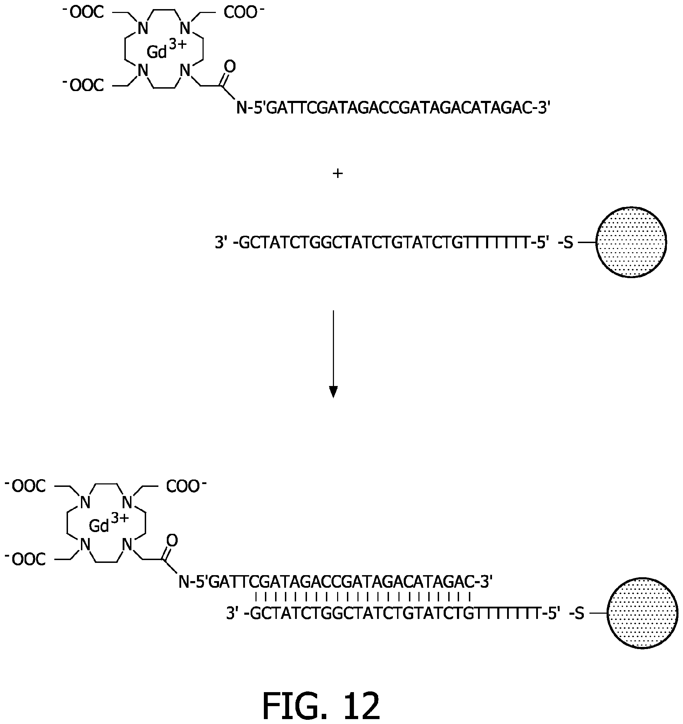

For electrospinning, polycaprolactone (PCL, 80 000 kD molecular weight) is used in a solution (8-20 % w/w PCL in a chloroform:methanol mixture of 5:1 to 7:1 mixing ratio). Into this solution, the colloidal gold coated with DNA-single-strands from step 1 is mixed in an amount of 0.01%). Electrospinning is then performed to build a network of fibers that can be shaped later (this process is described well in literature, *e.g.,* in Van Lieshout *et al.* (2006) and many others.
Later on, *i.e.,* after shaping, the DNA-single-strands modified with the Gd-complex are added to the complementary DNA-strands built into the scaffold by means of the Gold particles. Said binding can take place *in vitro* or *in vivo,* as described above. See Fig. 12 for an illustration of said binding process.

REFERENCES

Agard, N.J., Prescher, J.A., and Bertozzi, C.R., A Strain-Promoted [3 + 2] Azide-Alkyne Cycloaddition for Covalent Modification of Biomolecules in Living Systems. J. Am. Chem. Soc., 126, 46, 15046 - 15047, 2004
Grabar, K.C., R.G. Rreeman, M.B. Hommer, and M.J. Natan. 1995. Extended Oligothienylenevinylenes End-Capped with 1,4-Dithiafulvenyl -Donor Groups: Toward a Supramolecular Control of Effective Conjugation Length. Analyt. Chem. 67:735-743.
Saxon, E.; Bertozzi, C.R. Science 2000, 287, 2007
van Lieshout, M.I., C.M. Vaz, M.C. Rutten, G.W. Peters, and F.P. Baaijens, Electrospinning versus knitting: two scaffolds for tissue engineering of the aortic valve. JBiomater Sci Polym Ed. 17:77-89, 2006
Kuhnast, F. Hinnen, R. Boisgard, B. Tavitian, F. Dollé, Fluorine-18 labelling of oligonucleotides: Prosthetic labelling at the 5'-end using the N-(4-[18F] fluorobenzyl)-2-bromoacetamide reagent, Journal of Labelled Compounds and Radiopharmaceuticals 46 (12), 1093-1103, 2003

**Claims**

1. A method for the production of polymeric scaffold materials and/or scaffolds for tissue and/or organ engineering, said method comprising the addition of at least one anchoring unit for a labelling agent, to at least one scaffold material and/or to at least one scaffold, and the method further comprising the step of binding at least one labelling agent to at least one anchoring unit for a labelling agent, wherein the step of binding at least one labelling agent to at least one anchoring unit is carried out by means of a bio-orthogonal chemical reaction.

2. The method according to claim 1, wherein the polymeric scaffold materials and/or scaffold is produced by at least one method selected from the group consisting of

   a) electro spinning,
   b) rapid prototyping,
   c) knitting, and/or
   d) phase separation.

3. The method according to claim 1, wherein the scaffold material is a biodegradable material, and the anchoring unit is added to the reaction mixture prior to polymerization, wherein the anchoring unit is attached to the polymer from which the scaffold is formed.

4. The method according to claim 1, **characterized in that** the anchoring unit is attached after the forming of the scaffold, wherein the attachment of the anchoring unit is a covalent binding step.

5. The method according to claim 1, **characterized in that** the attachment of the anchoring unit is a non-covalent binding step.

6. The method according to claim 1, **characterized in that** the labelling agent is bound to the anchoring unit *in vivo.*

7. A scaffold material and/or a scaffold, obtainable by a method according to any of claims 1 - 6.

8. The scaffold according to claim 7, which is being used for the production of at least one tissue and/or organ selected from the group consisting of

   a) artificial heart valves,
   b) vascular grafts,
   c) skin,
   d) nervous tissue,
   e) organs,
   f) bladder,
   g) blood vessels,
   h) cartilage tissue, and/or
   i) bone tissue.

9. Use of a scaffold according to claim 7 for the manufacture of a tissue and/or organ.

10. A tissue and/or organ comprising a scaffold according to claim 7.

11. The tissue and/or organ according to claim 10, **characterized in that** said tissue and/or organ is selected from at least one of the group consisting of

    a) artificial heart valves,
    b) vascular grafts,
    c) skin,
    d) nervous tissue,

e) organs,
f) bladder,
g) blood vessels,
h) cartilage tissue, and/or
i) bone tissue.

**12.** A method of labelling a scaffold material and/or a scaffold for tissue and/or organ engineering according claim 7, said method comprising the step of binding at least one labelling agent to the anchoring unit, wherein at least one labelling agent is bound to the anchoring unit by means of a bio-orthogonal chemical reaction, wherein at least one labelling agent is bound to the anchoring unit *in vivo,* and said method comprising the step of releasing at least one labelling agent from the anchoring unit *in vivo.*

**Patentansprüche**

**1.** Verfahren zur Herstellung von polymeren Gerüstmaterialien und/oder Gerüsten für die Gewebe- und/oder Organtechnik, wobei das Verfahren die Zugabe von mindestens einer Verankerungseinheit für ein Kennzeichnungsmittel zu mindestens einem Gerüstmaterial und/oder zu mindestens einem Gerüst umfasst, und wobei das Verfahren weiter den Schritt des Bindens von mindestens einem Kennzeichnungsmittel an mindestens eine Verankerungseinheit für ein Kennzeichnungsmittel umfasst, wobei der Schritt des Bindens von mindestens einem Kennzeichnungsmittel an mindestens eine Verankerungseinheit mittels einer bioorthogonalen chemischen Reaktion durchgeführt wird.

**2.** Verfahren nach Anspruch 1, wobei das polymere Gerüstmaterial und/oder Gerüst durch mindestens ein Verfahren hergestellt wird, ausgewählt aus der Gruppe bestehend aus

a) Elektrospinnen,
b) Rapid Prototyping,
c) Stricken und/oder
d) Phasentrennung.

**3.** Verfahren nach Anspruch 1, wobei das Gerüstmaterial ein biologisch abbaubares Material ist und die Verankerungseinheit vor der Polymerisation dem Reaktionsgemisch zugegeben wird, wobei die Verankerungseinheit an dem Polymer befestigt ist, aus dem das Gerüst gebildet wird.

**4.** Verfahren nach Anspruch 1, **dadurch gekennzeichnet, dass** die Verankerungseinheit nach dem Bilden des Gerüsts befestigt wird, wobei die Befestigung der Verankerungseinheit ein kovalenter Bindungsschritt ist.

**5.** Verfahren nach Anspruch 1, **dadurch gekennzeichnet, dass** die Befestigung der Verankerungseinheit ein nichtkovalenter Bindungsschritt ist.

**6.** Verfahren nach Anspruch 1, **dadurch gekennzeichnet, dass** das Etikettiermittel *in vivo* an die Verankerungseinheit gebunden ist.

**7.** Gerüstmaterial und/oder Gerüst, erhältlich durch ein Verfahren nach einem der Ansprüche 1 - 6.

**8.** Gerüst nach Anspruch 7, das zur Herstellung von mindestens einem Gewebe und/oder Organ verwendet wird, ausgewählt aus der Gruppe bestehend aus

a) künstlichen Herzklappen,
b) Gefäßtransplantaten,
c) Haut,
d) Nervengewebe,
e) Organen,
f) Blase,
g) Blutgefäßen,
h) Knorpelgewebe und/oder
i) Knochengewebe.

9. Verwendung eines Gerüsts nach Anspruch 7 zur Herstellung eines Gewebes und/oder Organs.

10. Gewebe und/oder Organ, umfassend ein Gerüst nach Anspruch 7.

11. Gewebe und/oder Organ nach Anspruch 10, **dadurch gekennzeichnet, dass** das Gewebe und/oder Organ ausgewählt ist aus mindestens einem aus der Gruppe bestehend aus

> a) künstlichen Herzklappen,
> b) Gefäßtransplantaten,
> c) Haut,
> d) Nervengewebe,
> e) Organen,
> f) Blase,
> g) Blutgefäßen,
> h) Knorpelgewebe und/oder
> i) Knochengewebe.

12. Verfahren zum Kennzeichnen eines Gerüstmaterials und/oder eines Gerüsts für die Gewebe- und/oder Organtechnik nach Anspruch 7, wobei das Verfahren den Schritt des Bindens mindestens eines Kennzeichnungsmittels an die Verankerungseinheit umfasst, wobei mindestens ein Kennzeichnungsmittel mittels einer bio-orthogonalen chemischen Reaktion an die Verankerungseinheit gebunden ist, wobei mindestens ein Kennzeichnungsmittel an die Verankerungseinheit *in vivo* gebunden ist, und wobei das Verfahren den Schritt des Lösens mindestens eines Kennzeichnungsmittels von der Verankerungseinheit *in vivo* umfasst.

## Revendications

1. Procédé de production de matériaux d'ossature polymères et/ou d'ossatures pour la production de tissus et/ou d'organes, ledit procédé comprenant l'addition d'au moins une unité d'ancrage pour un agent de marquage, à au moins un matériau d'ossature et/ou à au moins une ossature et le procédé comprenant en outre l'étape de liaison d'au moins un agent de marquage à au moins une unité d'ancrage pour un agent de marquage, dans lequel l'étape de liaison d'au moins un agent de marquage à au moins une unité d'ancrage est effectuée au moyen d'une réaction chimique bio-orthogonale.

2. Procédé selon la revendication 1, dans lequel les matériaux d'ossature polymères et/ou les ossatures sont produits par au moins un procédé choisi dans le groupe constitué des suivants :

> a) électrofilage,
> b) prototypage rapide,
> c) tricotage et/ou
> d) séparation de phases.

3. Procédé selon la revendication 1, dans lequel le matériau d'ossature est un matériau biodégradable et l'unité d'ancrage est agitée au mélange réactionnel avant la polymérisation, dans lequel l'unité d'ancrage est fixée au polymère à partir duquel l'ossature est formée.

4. Procédé selon la revendication 1, **caractérisé en ce que** l'unité d'ancrage est fixée après la formation de l'ossature, dans lequel la fixation de l'unité d'ancrage est une étape de liaison covalente.

5. Procédé selon la revendication 1, **caractérisé en ce que** la fixation de l'unité d'ancrage est une étape de liaison non covalente.

6. Procédé selon la revendication 1, **caractérisé en ce que** l'agent de marquage est lié à l'unité d'ancrage *in vivo.*

7. Matériau d'ossature et/ou ossature susceptible(s) d'être obtenu(s) par un procédé selon l'une quelconque des revendications 1 à 6.

8. Ossature selon la revendication 7, qui est utilisée pour la production d'au moins un tissu et/ou un organe choisi(s) dans le groupe constitué des suivants :

a) valvules cardiaques artificielles,
b) greffes vasculaires,
c) peau,
d) tissu nerveux,
e) organes,
f) vessie,
g) vaisseaux sanguins,
h) tissu cartilagineux et/ou
i) tissu osseux.

9. Utilisation d'une ossature selon la revendication 7 pour la fabrication d'un tissu et/ou d'un organe.

10. Tissu et/ou organe comprenant une ossature selon la revendication 7.

11. Tissu et/ou organe selon la revendication 10, caractérisé(s) en ce que ledit tissu et/ou ledit organe est ou sont choisis parmi au moins l'un du groupe constitué des suivants :

a) valvules cardiaques artificielles,
b) greffes vasculaires,
c) peau,
d) tissu nerveux,
e) organes,
f) vessie,
g) vaisseaux sanguins,
h) tissu cartilagineux et/ou
i) tissu osseux.

12. Procédé de marquage d'un matériau d'ossature et/ou d'une ossature pour la production de tissus et ou d'organes selon la revendication 7, ledit procédé comprenant l'étape de liaison d'au moins un agent de marquage à l'unité d'ancrage, dans lequel au moins un agent de marquage est lié à l'unité d'ancrage au moyen d'une réaction chimique bio-orthogonale, dans lequel au moins un agent de marquage est lié à l'unité d'ancrage *in vivo* et ledit procédé comprenant l'étape de libération d'au moins un agent de marquage de l'unité d'ancrage *in vivo.*

FIG. 1

FIG. 2a

FIG. 2b

FIG. 3

FIG. 4a

FIG. 4b

EP 2 367 576 B1

anchoring unit --5'-ATGCAATGCTTAGTACTCGAAATG-3'
                 | | | | | | | | | | | | | | | | | | | | | | | |
              3'-TACGTTACGAATCATGAGCTTTAC-5'-- labelling agent

# FIG. 5a

anchoring unit --5'-ATCTGACTCGAAAT-3'
              | | | | | | | |
          3'-TAGACTGA-5'

                5'-TAGACTGA-3'
                | | | | | | | |
        3'-GCTTTAATCTGACT-5'-- labelling agent

# FIG. 5b

anchoring unit ------5'-ATGCAATGCTTAGTCCTCTGACTCGAAATG-3'
                | | | | | | | |                          | | | | | | | |
labelling agent 1 --3'-TACGTTAC-5'              3'-AGCTTTAC-5'--- labelling agent 2

FIG. 6a

anchoring unit ------5'-ATGCAATGCTTAGTGC-(X)ₙ -CCTCTGACTCGAAATG-3'
labelling agent 1 --3'-TACGTTACGAAT-5'              3'-TGAGCTTTAC-5'--- labelling agent 2

FIG. 6b

anchoring unit ------5'-ATGCAATGCTTAGTCCTCTGACTCGAAATGC-3'
labelling agent 1 --3'-TACGTT-5'    3'-AGGACA-5'    3'-TTACG-5'--- labelling agent 3

labelling agent 2

FIG. 6c

anchoring unit ------5'-ATGCAATGCTTA- spacer -ACTCGAAATG-3'
labelling agent 1 --3'-TACGTTACGAAT-5'       3'-TGAGCTTTAC-5'--- labelling agent 2

FIG. 6d

# FIG. 7a

# FIG. 7b

FIG. 8

FIG. 9

3' -GCTATCTGGCTATCTGTATCTGTTTTTTT-5' -SH   +

3' -GCTATCTGGCTATCTGTATCTGTTTTTTT-5' -S

# FIG. 10

FIG. 11

FIG. 12

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20060204445 A
- WO 2007039858 A2
- US 20080075661 A1
- WO 2007110811 A2
- WO 2007039864 A
- US 5523232 A
- EP 1332209 A


**Non-patent literature cited in the description**

- **AGARD, N.J. ; PRESCHER, J.A. ; BERTOZZI, C.R.** A Strain-Promoted [3 + 2] Azide-Alkyne Cycloaddition for Covalent Modification of Biomolecules in Living Systems. *J. Am. Chem. Soc.,* 2004, vol. 126 (46), 15046-15047
- **GRABAR, K.C. ; R.G. RREEMAN ; M.B. HOMMER ; M.J. NATAN.** Extended Oligothienylenevinylenes End-Capped with 1,4-Dithiafulvenyl -Donor Groups: Toward a Supramolecular Control of Effective Conjugation Length. *Analyt. Chem.,* 1995, vol. 67, 735-743
- **SAXON, E. ; BERTOZZI, C.R.** *Science,* 2000, vol. 287, 2007
- **VAN LIESHOUT, M.I. ; C.M. VAZ ; M.C. RUTTEN ; G.W. PETERS ; F.P. BAAIJENS.** Electrospinning versus knitting: two scaffolds for tissue engineering of the aortic valve. *JBiomater Sci Polym Ed.,* 2006, vol. 17, 77-89
- **KUHNAST, F. ; HINNEN, R. ; BOISGARD, B. ; TAVITIAN, F. DOLLÉ.** Fluorine-18 labelling of oligonucleotides: Prosthetic labelling at the 5'-end using the N-(4-[18F] fluorobenzyl)-2-bromoacetamide reagent. *Journal of Labelled Compounds and Radiopharmaceuticals,* 2003, vol. 46 (12), 1093-1103